# EUROPEAN PATENT SPECIFICATION

(11) **EP 2 596 783 B1**
(45) Date of publication and mention of the grant of the patent: **06.06.2018**
(21) Application number: 12186239.5
(22) Date of filing: 27.09.2012
(51) Int. Cl.: A61K 9/00, A61K 9/08

(54) **Pharmaceutical composition for the preparation of infusion solutions of antimicrobial preparations, its production process**
Pharmazeutische Zusammensetzung zur Herstellung von Infusionslösungen aus antimikrobiellen Zubereitungen, sowie Herstellungsverfahren
Composition pharmaceutique pour la préparation de solutions de perfusion de préparations antimicrobiennes, son procédé de fabrication

(30) Priority: 22.11.2011 RU 2011147170
(43) Date of publication of application: 29.05.2013
(73) Proprietor: Limonov, Viktor Lvovich, AD500 Andorra la Vella (AD)
(72) Inventor: Limonov, Viktor Lvovich, Moscow 115407 (RU); Gaidul, Konstantin Valentinovich, Novosibirsk (RU); Dushkin, Aleksandr Valerevich, Novosibirsk (RU)
(74) Representative: Ponti & Partners, S.L.P

(56) References cited:
- EP-A1- 2 476 419
- WO-A1-03/063877
- US-A- 3 252 859

## Description

This invention belongs to pharmaceutical preparations suitable for preparing solutions of injectable forms of antimicrobial pharmaceuticals, to the technologies of their compounding, it may be used in medicine and veterinary medicine when treating inflammatory infectious disease of different etiology, as well as in the pharmaceutical industry for producing substances and end dosage forms.

Traditionally, during many decades in the clinical practice for preparing solutions of the most antimicrobial (antibacterial and antifungal) preparations for intravenous injections and infusions thereof, the mostly used ingredients are water for injections and infusions, 0,9% solution of the sodium chloride, 5% solution of dextrose (glucose), less frequently 0,45% solution of the sodium chloride, 2% and 10% solution of dextrose, Ringer's solution, lactated Ringer's solution, solutions of potassium chloride and sodium chloride for intravenous infusions and some others, which by themselves have no antimicrobial action and do not have a potentiating action for the therapeutic efficacy of antimicrobial pharmaceuticals [1].

Due to this fact and taking into account that currently many clinically significant microbial strains have acquired more or less expressed resistibility to many antimicrobial preparations, the elaboration of new original approaches in order to essentially improve the antimicrobial activity and clinical efficacy of many antibacterial and antifungal pharmaceuticals when treating inflammatory infectious diseases is the urgent task of the modern experimental pharmacology and practical medicine.

Over the last years, is was discovered that the use of various nanoparticles as carriers for the delivery of different antibiotics directly to immune system cells providing for the anti-infectious protection of the organism (macrophages) in order to increase the intracellular concentration of these preparations and accordingly the intensification of their antimicrobial properties (which is in particular important with regard to microorganisms persistent in these cells: Chlamydia, mycoplasmas, mycobacteria, etc.), as well as for the stimulation of the macrophages' antibacterial activity and their additional recruitment into the infected tissues, is a very promising tendency of the development of new pharmaceutical technologies and new efficient methods of the antibiotic therapy [2, 3, 4, 5, 6, 7, 8, 9].

The aim of the present invention is to elaborate pharmaceutical compositions for preparing infusion solutions from pulverized injectable forms of antimicrobial preparations on the basis of sodium chloride, dextrose and colloidal silica (CS) having a potentiating action on the therapeutic efficacy of antibacterial and antifungal preparations in comparison with the traditional solvents (water for injections, 0,9% solution of the sodium chloride, 5% solution of dextrose, Ringer's solution and others, that are considered in the present invention as prototypes), and the process for the production thereof.

The technical result achieved during the realization of this invention is the intensification of the therapeutic efficacy of parenteral forms of antibacterial and antifungal preparations on the basis of use of nanoparticles and microparticles of colloidal silica.

Nanoparticles and microparticles of colloidal silica characterized by pharmacologically beneficial properties of biocompatibility, biodistribution, biodegradation and low toxicity, are able during the parenteral administration to serve as the carrier of antibiotics for the intracellular delivery to macrophages that are concentrated in the inflammatory tissues in the lungs, liver, kidneys, spleen, lymph nodes, heart, skin, bladder and other organs of mammals (what considerably increases the concentration of antibiotics at the intracellular level and in the infected tissues of the body), and considerably increase the antimicrobial activity of these cells of the immune systems (particularly, by means of stimulation of the nitric oxide generation, and activating the process of phagocytosis), thereby significantly increasing the therapeutic effect of antibacterial and antifungal pharmaceuticals [10, 11, 12, 13, 14, 15, 16, 17].

This problem has been solved by a group of inventions by creating a pharmaceutical composition for the preparation of infusion solutions of antibacterial and antifungal preparations as claimed in the appended claims 1 to 8.

### First variant

Pharmaceutical composition for the preparation of infusion solutions of antimicrobial preparations soluble in sterile water for injections, 0.45% and 0.9% sodium chloride solution, characterized in that it is prepared in a powder form, it contains sodium chloride and colloidal silica at the weight ratio equal to 4.5 or 9 : (1-5), respectively.

The new means of production of this pharmaceutical composition has been elaborated for the preparation of infusion solutions of antimicrobial preparations, including mixing of chloride with other components, distinguished by the fact that the powder sodium chloride is mixed with the powder-like colloidal silica at the weight ratio equal to 4.5 or 9 : (1-5), respectively, and the obtained mixture is subjected to mechanical treatment by means of impact and abrasive action on the substance to increase the mass fraction of fine-dispersed (less than 5 micron) particles of colloidal silica at least two times.

In order to prepare infusion solutions on the basis of use of the suggested pharmaceutical composition a single dosage of the dry powder of the antimicrobial preparation (soluble in the water for injections), indicated in the Instruction for use of said preparation is dissolved in 10 ml of the water for injections, afterwards the whole volume of the derived solution is transferred into the vial with the dry powder of the pharmaceutical composition indicated above, the same is intensively shaken for 2 or 3 minutes, after what the received suspension consisting of the solution of the antimicrobial preparation and the pharmaceutical composition is additionally dissolved in 50-100-200 ml of the 0.45% or 0.9% sodium chloride solution (depending on the content of the composition) and is injected intravenously as an infusion according to the requirements indicated in the Instruction for use of the antimicrobial preparation.

### Second variant

Pharmaceutical composition for the preparation of infusion solutions of antimicrobial preparations soluble in sterile water for injections, 2% and 5% dextrose solution characterized in that it is in the powder form, it contains dextrose and colloidal silica in the weight ratio equal to 20 or 50 : (1-5), respectively.

The process for the production of the pharmaceutical composition for the preparation of infusion solutions of antimicrobial preparations comprising mixing dextrose with other components characterized in that the dextrose in the powder form is mixed with the powder-like colloidal silica at the weight ratio of dextrose to colloidal silica equal to 20 or 50 : (1-5), respectively, and the obtained mixture is mechanically treated by means of impact and abrasive action until the mass fraction of fine-dispersed (less than 5 micron) particles of colloidal silica is increased at least twofold.

For the preparation of infusion solutions on the basis of using the suggested pharmaceutical composition the single dosage of the dry powder of the antimicrobial preparation (soluble in the water for injections) indicated in the Instruction for use is dissolved in 10 ml of the water for injection, after that the whole volume of the obtained solution is transferred into the vial with the dry powder of the pharmaceutical composition indicated above, the same is intensively shaken for 2 or 3 minutes, after that the received suspension consisting of the solution of the antimicrobial preparation and the pharmaceutical composition is additionally dissolved in 50-100-200 ml of the 2% or 5% dextrose solution (depending on the content of the composition) and is injected intravenously as an infusion according to the requirements indicated in the antimicrobial preparation prescribing information.

The therapeutic efficacy of antimicrobial preparations when using the proposed pharmaceutical composition is increased if the obtained mixture (sodium chloride + colloidal silica or dextrose + colloidal silica) is subjected to mechanical treatment by impact and abrasive action so that the contents of colloidal silica particles with the size ≤ 5 µm is at least 35%.

For the preparation of pharmaceutical compositions we used pharmaceutically applicable crystalline powder of sodium chloride and dextrose crystalline powder provided by the Russian producer of pharmaceuticals «ABOLmed» LLC along with the antimicrobial preparations (amoxycillin+clavulanate, aztreonam, cefotaxime, ceftriaxone, ceftazidime, cefopera-zone+sulbactam, cefepime, meropenem, amikacin sulfate, azithromycin, vancomycin, capreomycin, fosfomycin and voriconazole). As colloidal silica we used the pharmaceutically applicable AEROSIL 200 (generic name: colloidal silica) produced by «Evonik Degussa Corporation» (Germany) consisting of round shape nonporous silica nanoparticles (average diameter of 7-40 nm) joined into microparticles of non-regular form with the size < 100 um.

The choice of the make-up of the composition is based on the phenomenon of the reciprocal sorption of the molecules of antibacterial and antifungal preparations by nanoparticles and microparticles of colloidal silica, and the decrease of the size of the colloidal silica microparticles in case of the mechanical activation of the mixtures thereof with the crystalline powder of sodium chloride and dextrose crystalline powder by means of intensive mechanical impact and abrasive action.

The adding of the colloidal silica to the proposed compositions in the indicated weight ratios was defined experimentally on laboratory mice following the criterion of the maximum potentiating action onto the therapeutic efficacy of antimicrobial preparations with the minimum probability of the side effects.

The claimed process for the production of the above-indicated pharmaceutical compositions by means of mechanical activation of the powder blend of sodium chloride or dextrose with colloidal silica by intensive mechanical rubbing impact allows in comparison with other known means to increase the proportion of colloidal silica fine particles with the size ≤ 5 µm on which the molecules of antimicrobial preparations are adsorbed and that are actively phagocytized by macrophages [18].

For this purpose the mixture of the indicated agents (sodium chloride + colloidal silica or dextrose + colloidal silica) are subject to mechanical activation by means of intensive impact and abrasive action until the weight ratio of the colloidal silica fine powder fraction (≤ 5 um) is increased at least twofold.

The obtained powder pharmaceutical compositions are used for the production of infusion solutions consisting of fine particles of colloidal silica with the molecules of various antimicrobial preparations inversely adsorbed on their surface, soluble in the sterile water for injections.

For obtaining the compositions we used a mechanochemical approach consisting in the treatment of the mixture of the solid ingredients by intensive mechanical impact, that is pressure and shearing realized preferentially in various mills performing impacting rubbing influence on substances. The mixture of solid powder substances (sodium chloride + colloidal silica or dextrose + colloidal silica) is subject to mechanical activation in drum mills. The used way of obtaining compounds allows for achieving the full homogeneity of powder components in comparison with mixtures obtained by simple mixing of components, or by evaporation of their solutions and, as a result, ensures a high pharmacological activity of the pharmaceutical composition.

As a quantitative criterion of the minimally required portion of mechanical action it is convenient to use the method of granulometry of the suspension of the obtained composition. Herein it is necessary that the weight content of colloidal silica particles does not exceed 5 µm that may be measured by means of laser photometry, should increase at least twofold. The mechanical treatment of powder compounds is performed in rotary, vibratory or planetary mills. As grinding bodies it is possible to use balls, pivots, etc.

The pharmacological trials of the obtained compositions on laboratory animals (mice) have shown that the declared composition produced by the declared means has an express potentiating action onto the therapeutic efficiency of antimicrobial (antibacterial and antifungal) preparations when treating the bacterial sepsis induced by *Staphylococcus aureus, Escherichia coli* and *Pseudomonas aeruginosa,* as well as mycotic sepsis induced by *Candida albicans,* in comparison with usual solvents of antimicrobial medications.

Therefore, the use of the declared pharmaceutical compositions and their production process provide for the following advantages:
1) clinically significant increase of efficiency and quality of antimicrobial therapy of malignant contagious and inflammatory diseases, mortality decrease;
2) ecological safety, wastelessness and cost-efficiency of the technology of the pharmaceutical production.

The claimed invention is illustrated by examples listed below.

### Example 1. Production of the solid composition NaCl:colloidal silica.

The mixture of sodium chloride and colloidal silica in weight ratios 4.5:1, 4.5:2, 4.5:5, 9: 1, 9:2 and 9:5 is treated for 1, 2 or 4 hours in the drum rotary mill.

The analysis of the granulometric content of water suspensions of the particles of initial colloidal silica, as well different variations of compositions with NaCl was performed on the laser analyzer of particle dimensions Microsizer-201a produced by «VA Instalt», Russia. From 1g to 5g of the tested powder was placed into the sample preparation module (liquid volume of 150 sm³) in the quantity sufficient for achieving 70-75 % of optical transmission through the cuvet. The measuring was performed after mixing for one or two minutes with the simultaneous treatment of the suspension for the agglomerations' destruction. The data treatment was performed according to the calculation program embedded into the analyzer. The results were presented in the form of histograms of weight distribution by particle dimensions.

For defining the number of antimicrobial preparations sorbed by colloidal silica particles, 0.5g of antibiotic substance (by active matter) was dissolved in 5 cm³ water for injections. Thereafter, the known quantity of dry compositions NaCl : Colloidal silica was suspended in the fresh antibiotic solution, the obtained suspension was centrifuged during 30 minutes at a speed of 12000 rpm, the supernatant liquid was poured carefully, the residue of Colloidal silica was suspended again in the same quantity of water for injections. The concentration of antibiotic desorbing into the aqueous phase was defined by the HPLC method. Thereafter, the procedure of precipitating and suspending was repeated. The quantity of the sorbed antibiotic was calculated from the total defining of the antibiotic quantity desorbing from the colloidal silica residue.

The obtained data of the granulometric composition and sorption rate are shown in Table 1. As it follows from the obtained data, the chosen conditions for producing the claimed composition allow increasing the proportion of the colloidal silica fine powder fraction (with particles size ≤ 5 um) at least twofold and attain the binding degree of antimicrobial preparations molecules by colloidal silica particles at least by 40%.

**Table 1.**

| Granulometric data of water suspensions of composition and solution of antimicrobial preparations produced on the basis of use of the composition; the sorption rate of the preparation by colloidal silica particles | | |
|---|---|---|
| Content of the composition and solutions of antimicrobial preparations | Size and content % of colloidal silica particles | Sorption rate of the antimicrobial prepara-tion by colloidal silica particles (%) |
| | % ≤5 um | |
| Initial Colloidal silica | 15.2 | - |
| NaCl: Colloidal silica (4.5:1; mechanical activation 1 hour) | 38.5 | - |
| NaCl: Colloidal silica (4.5:2; mechanical activation 2 hours) | 41.3 | - |
| NaCl: Colloidal silica (4.5:5; mechanical activation 4 hours) | 39.2 | - |
| NaCl: Colloidal silica (9:1; mechanical activation 1 hour) | 37.7 | - |
| NaCl: Colloidal silica (9:2; mechanical activation 2 hours) | 43.9 | - |
| NaCl: Colloidal silica (9:5; mechanical activation 4 hours) | 35.8 | - |
| Ceftriaxone/NaCl : Colloidal silica (4.5:1; mechanical activation 1 hour) | 43.5 | 45.3 |
| Ceftriaxone/NaCl: Colloidal silica (4.5:2; mechanical activation 2 hours) | 45.4 | 47.8 |
| Ceftriaxone/NaCl: Colloidal silica (4.5:5; mechanical activation 4 hours) | 42.1 | 49.5 |
| Cefotaxime/NaCl: Colloidal silica (9:1; mechanical activation 1 hour) | 37.8 | 41.6 |
| Cefotaxime/NaCl: Colloidal silica (9:2; mechanical activation 2 hours) | 41.2 | 51.4 |
| Ceftazidime/NaCl: Colloidal silica (9:5; mechanical activation 2 hours) | 36.7 | 46.3 |
| Cefepime/NaCl: Colloidal silica (9:2; mechanical activation 2 hours) | 38,3 | 44,5 |
| Amikacin sulfate/NaCl: Colloidal silica (9:2; mechanical activation 2 hours) | 40,2 | 43,7 |
| Azithromycin/NaCl: Colloidal silica (9:2; mechanical activation 2 hours) | 39,1 | 51,9 |
| Vancomycin/NaCl: Colloidal silica (9:5; mechanical activation 2 hours) | 42,9 | 50,6 |
| Meropenem/NaCl: Colloidal silica (9:2; mechanical activation 2 hours) | 36,7 | 43,9 |
| Voriconazole/NaCl: Colloidal silica (9:2; mechanical activation 2 hours) | 37,5 | 41,7 |
| Capreomycin/NaCl: Colloidal silica (9:5; mechanical activation 2 hours) | 40,1 | 49,9 |

### Example 2. Production of the solid composition

Dextrose : Colloidal silica. The mixture of dextrose and colloidal silica in weight ratios 20:1, 20:2, 20:5, 50:1, 50:2 and 50:5 is processed for 1, 2 or 4 hours in the drum rotary mill.

The measurement of the granulometric content of water suspensions of the colloidal silica and the sorption rate of antibiotics was performed following the methods described in example 1. The obtained data are shown in Table 2. It follows from the data that the claimed method of preparation of the offered composition also allows increasing at least twofold the proportion of the colloidal silica fine powder fraction (with particles size ≤ 5 um) and ensuring the binding degree molecules of antimicrobial preparations by colloidal silica particles at least by 40%.

**Table 2.**

| Granulometric data of water suspensions of composition and solutions of antimicrobial preparations produced on the basis of use of the composition; the sorption rate of said preparations by colloidal silica particles | | |
|---|---|---|
| Content of the composition and solutions of antimicrobial preparations | Size and content % of colloidal silica particles | Sorption rate of the antimicrobial preparation by colloidal silica particles (%) |
| | % ≤5 um | |
| Initial Colloidal silica | 15.2 | - |
| Dextrose:Colloidal silica (20:1; mechanical activation 1 hour) | 39.1 | - |
| Dextrose:Colloidal silica (20:2; mechanical activation 2 hours) | 42.3 | - |
| Dextrose:Colloidal silica (20:5; mechanical activation 4 hours) | 41.2 | - |
| Dextrose:Colloidal silica (50:1; mechanical activation 1 hour) | 42.7 | - |
| Dextrose:Colloidal silica (50:2; mechanical activation 2 hours) | 39.9 | - |
| Dextrose:Colloidal silica (50:5; mechanical activation 4 hours) | 40.7 | - |
| Ceftriaxone/Dextrose:Colloidal silica (20:1; mechanical activation 1 hour) | 43.5 | 41.3 |
| Ceftriaxone/Dextrose:Colloidal silica (20:2; mechanical activation 2 hours) | 48.4 | 47.8 |
| Ceftriaxone/Dextrose:Colloidal silica (20:5; mechanical activation 4 hours) | 42.1 | 51.5 |
| Cefotaxime/Dextrose:Colloidal silica (50:1; mechanical activation 1 hour) | 41.8 | 40.6 |
| Cefotaxime/Dextrose:Colloidal silica (50:2; mechanical activation 2 hours) | 44.2 | 51.4 |
| Cefotaxime/Dextrose:Colloidal silica (50:5; mechanical activation 4 hours) | 46.7 | 66.3 |
| Ceftazidime/Dextrose:Colloidal silica (50:2; mechanical activation 2 hours) | 37.9 | 47.8 |
| Cefepime/Dextrose:Colloidal silica (50:2; mechanical activation 2 hours) | 42.1 | 44.9 |
| Azithromycin/Dextrose:Colloidal silica (50:2; mechanical activation 2 hours) | 41.8 | 55.7 |
| Vancomycin/Dextrose:Colloidal silica (50:5; mechanical activation 2 hours) | 36.9 | 50.9 |
| Meropenem/Dextrose:Colloidal silica (50:2; mechanical activation 2 hours) | 40.5 | 78.5 |
| Voriconazole/Dextrose:Colloidal silica (50:2; mechanical activation 2 hours) | 35.1 | 47.1 |
| Amikacin sulfate/Dextrose:Colloidal silica (50:2; mechanical activation 2 hours) | 43.6 | 52.3 |

### Example 3. Determination of the therapeutic efficiency of antimicrobial preparations the solutions of which (for intravenous injection) are prepared on the basis of use of the pharmaceutical composition.

Beta-lactam antibiotics have been tested (amoxycillin+clavulanate, cefotaxime, ceftriaxone, cefoperazone+sulbactam, ceftazidime, cefepime, aztreonam, meropenem), of mac-rolides (azithromycin), of aminoglycosides (amikacin sulfate), of glycopeptides (vancomycin), of antifungal agent (voriconazole), as well as of fosfomycin.

In order to determine the therapeutic efficacy of antimicrobial agents as a basis we took experimental models of sepsis and the method of statistical treatment of the obtained results (χ²) according to [19, 20].

Microorganisms: *Staphylococcus aureus* (ATCC Nº 25923 F-49), *Escherichia coli* (ATCC Nº25922 F-50), *Pseudomonas aeruginosa* (ATCC Nº27853 F-51), *Candida albicans* (ATCC Nº 24433).

Animals: the experiments were carried out on hybrid mice (CBA x C₅₇Black/₆)CBF₁ according to the «Regulations on the work with test animals» (USSR Ministry of health order supplement Nº 755 from 12.08. 1977).

### Experimental sepsis models

The mice have been injected intravenously 0,8ml of daily suspension of culture *P*. *aeruginosa* in a dose 5x10⁸ CFU/mouse or daily suspension of culture *S. aureus* in a dose 10¹⁰ CFU/mouse or *E. coli* daily culture suspension with a dosage 8x10⁸ CFU/mouse or daily suspension of culture *Candida albicans* with a dose 10¹⁰ CFU/mouse. The control group of mice has been injected the 0.9% solution of NaCl or 5% dextrose solution at the amount 0.8 ml.

In a day after being infected the test mice have been daily during 3 days intravenous injected with the germicides indicated above dissolved in 0.9% solution of NaCl or in 5% dextrose solution, as well as their solutions prepared on the basis of using the pharmaceutical composition (as it is described here above).

All beta-lactams were injected daily at the amount of 0.2 mg/mouse, amikacin sulfate at the amount of 2 mg/mouse daily, vancomycin at the amount of 1 mg/mouse daily, fosfomycin at the amount of 2 mg/mouse daily, voriconazole at the amount of 0.1 mg/mouse daily, contained in 0.5 ml of solution. Following the same scheme the control group was injected the 0.9% of NaCl solution or the 5% dextrose solution, as well as water solutions of pharmaceutical compositions at the amount of 0.5 ml.

The efficacy of the antibacterial therapy was evaluated on the basis of the number of the survived mice on the seventh day after being infected [19, 20].

The obtained data shown in tables 3 and 4 reflect the results of three independent experiments (for each preparation research at least 30 test animals were used).

**Table 3.**

| Therapeutic efficacy of the antimicrobial preparations in the treatment of sepsis (solutions of the preparations have been prepared on the basis of c omposition NaCl:Colloidal silica) | | | | | |
|---|---|---|---|---|---|
| Tested antibiotics | Mice survival rate on the 7th day of infection * | | | | χ₂ |
| | *S. aureus* | *E. coli* | *P. aeruginosa* | *Candida albicans* | |
| 0,9% NaCl solution | 0% (0/31) | 0% (0/30) | 0% (0/32) | 0% (0/30) | - |
| Solution NaCl:Colloidal silica (9:2; mechanical activation 2 hours) | 0% (0/30) | 0% (0/34) | 0% (0/32) | 0% (0/31) | - |
| Amoxycillin+clavulanate/ 0,9% NaCl solution | 40.0% (12/30) | 41.9% (13/31) | - | -** | P<0.01 |
| Amoxycillin+clavulanate/ NaCl:Colloidal silica (9:2; mechanical activation 2 hours) | 83,9% (26/31) | 83,3% (25/30) | - | - | |
| Cefotaxime/0,9% NaCl solution | 43.7% (14/32) | 37.5% (12/32) | - | - | P<0.01 |
| Cefotaxime/NaCl: Colloidal silica (9:2; mechanical activation 2 hours) | 81.2% (26/32) | 86.7% (26/30) | - | - | |
| Cefoperazone+sulbactam / 0,9% NaCl solution | 43.3% (13/30) | 59.3% (19/32) | 46.6% (14/30) | - | P<0.01 |
| Cefoperazone+sulbactam / NaCl:Colloidal silica (9:2; mechanical activation 2 hours) | 80.6% (25/31) | 93.5% (29/31) | 93.3% (28/30) | - | |
| Ceftazidime/0,9% NaCl solution | 38.7% (12/31) | 48.4% (15/31) | 46.7% (14/30) | - | P<0.01 |
| Ceftazidime/NaCl: Colloidal silica (9:2; mechanical activation 2 hours) | 78.1% (25/32) | 90.6% (29/32) | 87.0% (27/31) | - | |
| Cefepime/0,9% NaCl solution | 46.7% (14/30) | 58.1% (18/31) | 51.6% (16/31) | - | P<0.01 |
| Cefepime/NaCl: Colloidal silica (9:2; mechanical activation 2 hours | 83.3% (25/30) | 93.3% (28/30) | 90.0% (27/30) | - | |
| Aztreonam/0,9% NaCl solution | - | 70.0% (21/30) | 67.7% (21/31) | - | P<0.01 |
| Aztreonam/NaCl:Colloidal silica (9:2; mechanical activation 2 hours) | - | 93.9% (31/33) | 90.3% (28/31) | - | |
| Meropenem/0,9% NaCl solution | 70.9% (22/31) | 73.8% (31/42) | 71.8% (23/32) | - | P<0.01 |
| Meropenem/NaCl:Colloidal | 90.9% | 95.2% | 94.1% (32/34) | - | |
| silica (9:2; mechanical activation 2 hours) | (30/33) | (40/42) | | | |
| Azithromycin/0,9% NaCl solution | 43.3% (13/30) | - | - | - | P<0.01 |
| Azithromycin/NaCl: Colloidal silica (9:2; mechanical activation 2 hours) | 90.0% (27/30) | - | - | - | |
| Vancomycin/0.9% NaCl solution | 71.4% (30/42) | - | - | - | P<0.01 |
| Vancomycin/NaCl: Colloidal silica (9:2; mechanical activation 2 hours) | 97.5% (39/40) | - | - | - | |
| Amikacin sulfate/0.9% NaCl solution | - | 48.3% (15/31) | - | - | P<0.01 |
| Amikacin sulfate/ NaCl: Colloidal silica (9:2; mechanical activation 2 hours) | - | 86.6% (26/30) | - | - | |
| Fosfomycin/0.9% NaCl solution | 36.7% (11/30) | 43.3 % (13/30) | 30.0% (9/30) | - | P<0.01 |
| Fosfomycin/NaCl: Colloidal silica (9:2; mechanical activation 2 hours) | 83.3% (25/30) | 86.7% (26/30) | 61.3% (19/31) | - | |
| Voriconazole/0.9% NaCl solution | - | - | - | 45.1% (14/31) | P<0.01 |
| Voriconazole/NaCl: Colloidal silica (9:2; mechanical activation 2 hours) | - | - | - | 90.3% (28/31) | |

| | | | | | |
|---|---|---|---|---|---|
| *- in % and absolute values (survival rate/infected animals). ** - tests were not conducted | | | | | |

**Table 4.**

| Therapeutic efficacy of the antimicrobial preparations in the treatment of sepsis (solutions of the preparations have been prepared on the basis of composition Dextrose:Colloidal silica) | | | | | |
|---|---|---|---|---|---|
| Tested antibiotics | Mice survival rate on the 7th day of infection * | | | | χ₂ |
| | *S. aureus* | *E. coli* | *P. aeruginosa* | *Candida albicans* | |
| 5% dextrose solution | 0% (0/31) | 0% (0/30) | 0% (0/32) | 0% (0/30) | - |
| Solution Dextrose:Colloidal silica (50:2; mechanical activation 2 hours) | 0% (0/30) | 0% (0/31) | 0% (0/30) | 0% (0/30) | - |
| Ceftriaxone/5% dextrose solution | 40.6% (13/32) | 45.2% (14/31) | - | -** | P<0.01 |
| Ceftriaxone/ Dextrose:Colloidal silica (50:2; mechanical activation 2 hours) | 83.9% (26/31) | 90.0% (27/30) | - | - | |
| Cefotaxime/ 5% dextrose solution | 42.8% (15/35) | 43.7% (14/32) | - | - | P<0.01 |
| Cefotaxime/ Dextrose:Colloidal silica (50:2; mechanical activation 2 hours) | 84.4% (27/32) | 81.2% (26/32) | - | - | |
| Ceftazidime/ 5% dextrose solution | 40.0% (12/30) | 53.3% (16/30) | 46.8% (15/32) | - | P<0.01 |
| Ceftazidime/ Dextrose:Colloidal silica (50:2; mechanical activation 2 hours) | 86.7% (26/30) | 93.3% (28/30) | 87.0% (27/31) | - | |
| Cefepime/ 5% dextrose solution | 56.7% (17/30) | 54.4% (17/31) | 50.0% (15/30) | - | P<0.01 |
| Cefepime/ Dextrose:Colloidal silica (50:2; mechanical activation 2 hours) | 90.0% (27/30) | 93.7% (30/32) | 93.5% (29/31) | - | |
| Azithromycin/ 5% dextrose solution | 43.3% (13/30) | - | - | - | P<0.01 |
| Azithromycin/ Dextrose:Colloidal silica (50:2; mechanical activation 2 hours) | 80.6% (25/31) | - | - | - | |
| Vancomycin/ 5% dextrose solution | 77.5% (31/40) | - | - | - | P<0.01 |
| Vancomycin/ Dextrose:Colloidal silica (50:2; mechanical activation 2 hours) | 95.0% (38/40) | - | - | - | |
| Meropenem/ 5% dextrose solution | 73.8% (31/42) | 78.0% (32/41) | 74.4% (32/43) | - | P<0.01 |
| Meropenem/Dextrose:Colloidal silica (50:2; mechanical activation 2 hours) | 95.1% (39/41) | 95.0% (38/40) | 95.2% (40/42) | - | |
| Amikacin sulfate/ 5% dextrose solution | - | 46.7% (14/30) | - | - | P<0.01 |
| Amikacin sulfate/ Dextrose:Colloidal silica (50:2; mechanical activation 2 hours) | - | 83.3% (25/30) | - | - | |
| Fosfomycin/ 5% dextrose solution | 43.7% (14/32) | 46.7% (14/30) | 35.2% (15/34) | - | P<0.01 |
| Fosfomycin/ Dextrose:Colloidal silica (50:2; mechanical activation 2 hours) | 87.5% (28/32) | 90.0% (27/30) | 85.3% (29/34) | - | |
| Voriconazole/ 5% dextrose solution | - | - | - | 46.7% (14/30) | P<0.01 |
| Voriconazole/ Dextrose:Colloidal silica (50:2; mechanical activation 2 hours) | - | - | - | 93.5% (29/31) | |

| | | | | | |
|---|---|---|---|---|---|
| *- in % and absolute values (survival rate/infected animals). ** - tests were not conducted | | | | | |

As you may see from Tables 3 and 4, all the suggested pharmaceutical compositions for preparing injections of all the tested antimicrobial preparations containing the finely dispersed powder of nanostructured colloidal silica, faithfully increase their therapeutic efficiency when treating overwhelming sepsis of test animals, provoked by *Staphylococcus aureus, Escherichia coli, Pseudomonas aeruginosa* and *Candida albicans.*

Therefore, on the basis the obtained data, a conclusion may be made that the offered pharmaceutical compositions for preparing solutions of antibacterial and antifungal preparations for intravenous infusions (NaCl : Colloidal silica and Dextrose : Colloidal silica) have a significant clinically significant potentiating action on their therapeutic potential when treating inflammatory infectious disease, in comparison with traditional solvents (prototypes of the invention).

### Used literature

1. Kucers' The use of antibiotics// By M.L.Grauson, S.M.Crowe, J.S.McCarthy et al. 6th ed, 2 vols, 3000 pp. London, UK, Hodder Education/ASM Press, 2010.
2. Abeylath S.C., Turos E. Drug delivery approaches to overcome bacterial resistance to β-lactam antibiotics // Expert Opinion on Drug Delivery. - 2008. - Vol.5. - P.931-949.
3. Bastus N.G., Sanchez-Tillo E., Pujals S. et al. Peptides conjugated to gold nanoparticles induce macrophage activation // Molecular Immunology. - 2009. - Vol.46. - P.743-748.
4. Pinto-Alphandary H., Andremont A., Couvreur P. Targeted delivery of antibiotics using liposomes and nanoparticles: research and applications // International Journal of Antimicrobial Agents. - 2000. - Vol.13. - P.155-168.
5. Ulbrich W., Lamprech A. Targeted drug-delivery approaches by nanoparticulate carriers in the therapy of inflammatory diseases // Journal Royal Society Interface. - 2010. - Vol.7, Suppl. 1. - P.S55-S66.
6. Rosemary M.J., MacLaren I., Pradeep T. Investigation of antibacterial properties of ciprofloxacin@SiO2. // Langmuir. - 2006. - Vol.22. - P.10125-10129.
7. Rai A., Prabhune A., Perry C.C. Antibiotic mediated synthesis of gold nanoparticles with potent antimicrobial activity and their application in antimicrobial coatings // Journal of Materials Chemistry. - 2010. - Vol.20. - P.6789-6798.
8. Zolnik B.S., Gonzalez-Fernandez A., Sadrieh N., Dobrovolskaia V. Minireview: Nanoparticles and the immune system // Endocrinology. - 2010. - Vol.151. - P.458-465.
9. Pinto-Alphandary H., Balland O., Laurent M. et al. Intracellular visualization of ampicillin-loaded nanoparticles in peritoneal macrophages infected in vitro with Salmonella ty-phimurium // Pharmaceutical Research. - 1994. - Vol.11. - P.38-46.
10. Park J-H., Gu L., Maltzahn G. et al. Biodegradable luminescent porous silicon nanoparticles for in vivo applications // Nature Materials. - 2009. - Vol.8. - P.331-336.
11. Hetrick E.M., Shin J.H., Stasko N.A. et al. Bactericidal efficacy of nitric oxide-releasing silica nanoparticles// ACS Nano. - 2008. - Vol.2. - P.235-246.
12. Pernis B. Silica and the immune system // Acta Biomed. - 2005. - Vol.76, Suppl. 2.-P.38-44.
13. Tasciotti E., Liu X., Bhavane R. Et et al. Mesoporous silicon particles as a multistage delivery system for imaging and therapeutic applications // Nature Nanotechnology. - 2008. - Vol.3. - P.151-157.
14. Seleem M.N., Munusamy P., Ranjan A et al. Silica-antibiotic hybrid nanoparticles for targeting intracellular pathogens // Antimicrobial Agents and Chemotherapy. - 2009. - Vol.53. - P.4270-4274.
15. Chuiko A., Pentyuk A., Shtat'ko E., Chuiko N. Medical aspects of application of highly disperse amorphous silica // Surface Chemistry in Biomedical and Environmental Science. Edited by J.P.Blitz and V. Gun'ko.II. Mathematics, Physics and Chemistry. - 2006. - Vol.228. - P.191-204.
16. Waters K.M., Masiello L.M., Zangar R.C. et al. Macrophage responses to silica nanoparticles are highly conserved across particle sizes // Toxicological Sciences. - 2009. - Vol.107. - P. 553-569.
17. Lucarelli M., Gatti A.M., Savarino G. et al. Innate defence functions of macrophages can be biased by nano-sized ceramic and metallic particles // European Cytokine Network. - 2004. - Vol.15. - P.339-346.
18. Hamilton R.F., Thakur S.A., Mayfair J.K., Holian A. MARCO mediates silica uptake and toxicity in alveolar macrophages from C57BL/6 mice // Journal Biological Chemistry. - 2006. - Vol.281. - P. 34218-34226.
19. Eckhardt C., Fickweiler K., Schaumann R. et al. Therapeutic efficacy of moxifloxacin in a murine model of severe systemic mixed infection with E.coli and B.fragilis // Anaerobe. - 2003. - Vol.9. - P.157-160.
20. Schaumann R., Blatz R., Beer J. et al. Effect of moxifloxacin versus imipenem/cilastatin treatment on the mortality of mice infected intravenously with different strains of Bacteroides fragilis and Escherichia coli // Journal of Antimicrobial Chemotherapy. - 2004. - Vol.53. - P.318-324.

## Claims

1. A powder composition for the preparation of infusion solutions of antimicrobial preparations soluble in sterile water for injection, 0.45% and 0.9% solution of sodium chloride, **characterized in that** the powder composition comprises powder sodium chloride and a mechano-chemical activated powder of colloidal silica and sodium chloride, wherein the sodium chloride and the colloidal silica are at a weight ratio of 4.5:1 to 4.5:5 or 9:1 to 9:5, respectively, and the mechano-chemical activated powder is activated by intensive mechanical impact and abrasive action and comprises at least 35% of colloidal silica particles with a size ≤ 5 µm.

2. A powder composition for the preparation of infusion solutions of antimicrobial preparations soluble in sterile water for injection, 2% and 5% dextrose solution, **characterized in that** the powder composition comprises dextrose in powder form and a mechano-chemical activated powder of colloidal silica and dextrose, wherein the dextrose and the colloidal silica are at a weight ratio of 20:1 to 20:5 or 50:1 to 50:5, respectively, and the mechano-chemical activated powder is activated by intensive mechanical impact and abrasive action and comprises at least 35% of colloidal silica particles with a size ≤ 5 µm.

3. Powder composition according to claims 1 or 2, **characterized in that** the colloidal silica consists of silica nanoparticles having an average diameter of from 7 nm to 40 nm joined into microparticles having a size of less than 100 µm.

4. A method for producing a powder composition for the preparation of infusion solutions of antimicrobial preparations, said method including mixing sodium chloride with other components, **characterized in that** sodium chloride in the powder form is mixed with powder-like colloidal silica at a weight ratio of sodium chloride to colloidal silica of 4.5:1 to 4.5:5 or 9:1 to 9:5, respectively, and the obtained mixture is mechanically activated by impact and abrasive action until the mass fraction of fine-dispersed particles of colloidal silica is increased at least twofold, thereby comprising at least 35% of colloidal silica particles with a size ≤ 5 µm to providing the powder composition comprising a mechano-chemical activated powder of colloidal silica mixed with powder sodium chloride.

5. A method for producing a powder composition for the preparation of infusion solutions of antimicrobial preparations, said method including mixing dextrose with other components, **characterized in that** dextrose in the powder form is mixed with powder-like colloidal silica at a weight ratio of dextrose to colloidal silica of 20:1 to 20:5 or 50:1 to 50:5, respectively, and the obtained mixture is mechanically activated by impact and abrasive action until the mass fraction of fine-dispersed particles of colloidal silica is increased at least twofold, thereby comprising at least 35% of colloidal silica particles with a size ≤ 5 µm to provide the powder composition comprising a mechano-chemical activated powder of colloidal silica mixed with powder dextrose.

6. Method for producing a powder composition for the preparation of infusion solutions of antimicrobial preparations according to claims 4 or 5, **characterized in that** the colloidal silica consists of silica nanoparticles having an average diameter of from 7 nm to 40 nm joined into microparticles having a size of less than 100 µm.

7. A method for preparing an infusion solution of antimicrobial preparations using a powder composition comprising powder sodium chloride or powder dextrose according to any one of claims 1 to 3, **characterized in that** the method comprises:
- selecting an antimicrobial agent to be injected in a mammal, being the antimicrobial agent in a dry form;
- dissolving the antimicrobial agent in sterile water to provide an antimicrobial solution;
- mixing the antimicrobial solution with the powder composition by intensively shaken to provide an antimicrobial solution for infusion; and
- diluting the prepared antimicrobial infusion solution either in 50 - 200 ml of 0.45% or 0.9% sodium chloride solution, respectively, or in 50 - 200 ml of 2% or 5% dextrose solution, respectively, in accordance with the powder composition comprising powder sodium chloride or powder dextrose, respectively, to provide an antimicrobial infusion solution preparation ready-to-use.

8. Method according to claim 7, **characterized in that** the antimicrobial infusion solution preparation ready-to-use comprises at least 40% of the antimicrobial agent sorbed at the mechano-chemical activated powder of colloidal silica.

## Patentansprüche

1. Eine Pulverzusammensetzung für die Zubereitung von Infusionslösungen antimikrobieller Zubereitungen, die in sterilem Wasser zur Injektion, 0,45 %-iger sowie 0,9 %-iger Natriumchloridlösung löslich sind, **dadurch gekennzeichnet, dass** die Pulverzusammensetzung pulverförmiges Natriumchlorid und ein mechano-chemisch aktiviertes Pulver aus kolloidalem Siliciumdioxid und Natriumchlorid aufweist, wobei das Natriumchlorid und das kolloidale Siliciumdioxid in einem Gewichtsverhältnis von 4,5:1 zu 4,5:5 beziehungsweise 9:1 zu 9:5 vorliegen, und das mechano-chemisch aktivierte Pulver durch eine intensive mechanische Einwirkung und eine abrasive Wirkung aktiviert wird und zumindest 35 % kolloidale Siliciumdioxidpartikel mit einer Größe von ≤ 5 µm aufweist.

2. Eine Pulverzusammensetzung für die Zubereitung von Infusionslösungen antimikrobiellen Zubereitungen, die in sterilem Wasser zur Injektion, 2 %-iger sowie 5 %-iger Dextroselösung löslich sind, **dadurch gekennzeichnet, dass** die Pulverzusammensetzung Dextrose in Pulverform und ein mechano-chemisch aktiviertes Pulver aus kolloidalem Siliciumdioxid und Dextrose aufweist, wobei die Dextrose und das kolloidale Siliciumdioxid in einem Gewichtsverhältnis von 20:1 bis 20:5 beziehungsweise 50:1 zu 50:5 vorliegen, und das mechano-chemisch aktivierte Pulver durch eine intensive mechanische Einwirkung und eine abrasive Wirkung aktiviert wird und zumindest 35 % kolloidale Siliciumdioxidpartikel mit einer Größe von ≤ 5 µm aufweist.

3. Pulverzusammensetzung gemäß Anspruch 1 oder 2, **dadurch gekennzeichnet, dass** das kolloidale Siliciumdioxid aus Siliciumdioxidnanopartikeln mit einem durchschnittlichen Durchmesser von 7 nm bis 40 nm besteht, die zu Mikropartikeln mit einer Größe von weniger als 100 µm zusammengefügt sind.

4. Ein Verfahren zum Erzeugen einer Pulverzusammensetzung für die Zubereitung von Infusionslösungen antimikrobieller Zubereitungen, wobei das Verfahren ein Mischen von Natriumchlorid mit anderen Komponenten umfasst, **dadurch gekennzeichnet, dass** Natriumchlorid in der Pulverform mit pulverähnlichem kolloidalem Siliciumdioxid bei einem Gewichtsverhältnis von Natriumchlorid zu kolloidalem Siliciumdioxid von 4,5:1 bis 4,5:5 beziehungsweise 9:1 bis 9:5 gemischt wird, und die erhaltene Mischung mechanisch durch eine Einwirkung und eine abrasive Wirkung aktiviert wird, bis der Massenanteil von fein dispergierten Partikeln aus kolloidalem Siliciumdioxid zumindest zwanzigfach erhöht ist, wodurch dieselbe zumindest 35 % kolloidale Siliciumdioxidpartikel mit einer Größe von ≤ 5 µm aufweist, um die Pulverzusammensetzung bereitzustellen, die ein mechano-chemisch aktiviertes Pulver aus kolloidalem Siliciumdioxid gemischt mit pulverförmigem Natriumchlorid aufweist.

5. Ein Verfahren zum Erzeugen einer Pulverzusammensetzung für die Zubereitung von Infusionslösungen antimikrobieller Zubereitungen, wobei das Verfahren ein Mischen von Dextrose mit anderen Komponenten umfasst, **dadurch gekennzeichnet, dass** Dextrose in der Pulverform mit pulverähnlichem kolloidalem Siliciumdioxid bei einem Gewichtsverhältnis von Dextrose zu kolloidalem Siliciumdioxid von 20:1 bis 20:5 beziehungsweise 50:1 bis 50:5 gemischt wird, und die erhaltene Mischung mechanisch durch eine Einwirkung und eine abrasive Wirkung aktiviert wird, bis der Massenanteil von fein dispergierten Partikeln aus kolloidalem Siliciumdioxid zumindest zwanzigfach erhöht ist, wodurch dieselbe zumindest 35 % kolloidale Siliciumdioxidpartikel mit einer Größe von ≤ 5 µm aufweist, um die Pulverzusammensetzung bereitzustellen, die ein mechano-chemisch aktiviertes Pulver aus kolloidalem Siliciumdioxid gemischt mit pulverförmiger Dextrose aufweist.

6. Verfahren zum Erzeugen einer Pulverzusammensetzung für die Zubereitung von Infusionslösungen aus antimikrobiellen Zubereitungen gemäß Anspruch 4 oder 5, **dadurch gekennzeichnet, dass** das kolloidale Siliciumdioxid aus Siliciumdioxidnanopartikeln mit einem durchschnittlichen Durchmesser von 7 nm bis 40 nm besteht, die zu Mikropartikeln mit einer Größe von weniger als 100 µm zusammengefügt sind.

7. Ein Verfahren zum Erzeugen einer Infusionslösung aus antimikrobiellen Zubereitungen unter Verwendung einer Pulverzusammensetzung, die pulverförmiges Natriumchlorid oder pulverförmige Dextrose aufweist, gemäß einem der Ansprüche 1 bis 3, **dadurch gekennzeichnet, dass** das Verfahren folgende Merkmale aufweist:
- Auswählen eines antimikrobiellen Mittels, das in ein Säugetier zu injizieren ist, wobei das antimikrobielle Mittel in einer trockenen Form vorliegt;
- Lösen des antimikrobiellen Mittels in sterilem Wasser, um eine antimikrobielle Lösung bereitzustellen;
- Mischen der antimikrobiellen Lösung mit der Pulverzusammensetzung durch intensives Schütteln, um eine antimikrobielle Lösung zur Infusion bereitzustellen; und
- Verdünnen der zubereiteten antimikrobiellen Infusionslösung entweder in 50 - 200 ml 0,45%-iger beziehungsweise 0,9 %-iger Natriumchloridlösung oder in 50 - 200 ml 2 %-iger beziehungsweise 5 %-iger Dextroselösung gemäß der Pulverzusammensetzung, die pulverförmiges Natriumchlorid oder pulverförmige Dextrose aufweist, um eine gebrauchsfertige antimikrobielle Infusionslösungszubereitung bereitzustellen.

8. Verfahren gemäß Anspruch 7, das **dadurch gekennzeichnet ist, dass** die gebrauchsfertige antimikrobielle Infusionslösungszubereitung zumindest 40 % des antimikrobiellen Mittels aufweist, das an dem mechano-chemisch aktivierten Pulver aus kolloidalem Siliciumdioxid sorbiert ist.

## Revendications

1. Composition de poudre pour la préparation de solutions pour perfusion de préparations antimicrobiennes solubles dans l'eau stérile pour injection, solution de chlorure de sodium à 0,45 % et 0,9 %, **caractérisée en ce que** la composition de poudre comprend du chlorure de sodium en poudre et une poudre activée par voie mécano-chimique de silice colloïdale et de chlorure de sodium, dans laquelle le chlorure de sodium et la silice colloïdale sont dans un rapport pondéral de 4,5/1 à 4,5/5 ou de 9/1 à 9/5, respectivement, et la poudre activée par voie mécano-chimique est activée par choc mécanique intense et action abrasive et comprend au moins 35 % de particules de silice colloïdale d'une taille ≤ 5 µm.

2. Composition de poudre pour la préparation de solutions pour perfusion de préparations antimicrobiennes solubles dans l'eau stérile pour injection, solution de dextrose à 2 % et 5 %, **caractérisée en ce que** la composition de poudre comprend du dextrose sous forme de poudre et une poudre activée par voie mécano-chimique de silice colloïdale et de dextrose, dans laquelle le dextrose et la silice colloïdale sont dans un rapport pondéral de 20/1 à 20/5 ou de 50/1 à 50/5, respectivement, et la poudre activée par voie mécano-chimique est activée par choc mécanique intense et action abrasive et comprend au moins 35 % de particules de silice colloïdale d'une taille ≤ 5 µm.

3. Composition de poudre selon les revendications 1 ou 2, **caractérisée en ce que** la silice colloïdale est constituée de nanoparticules de silice ayant un diamètre moyen de 7 nm à 40 nm regroupées en microparticules ayant une taille inférieure à 100 µm.

4. Procédé de production d'une composition de poudre pour la préparation de solutions pour perfusion de préparations antimicrobiennes, ledit procédé incluant le mélange de chlorure de sodium avec d'autres composants, **caractérisé en ce que** le chlorure de sodium sous forme de poudre est mélangé avec une silice colloïdale de type poudre en un rapport pondéral du chlorure de sodium à la silice colloïdale de 4,5/1 à 4,5/5 ou de 9/1 à 9/5, respectivement, et le mélange obtenu est activé mécaniquement par choc et action abrasive jusqu'à ce que la fraction massique des particules finement dispersées de silice colloïdale soit augmentée au moins par deux, comprenant ainsi au moins 35 % de particules de silice colloïdale d'une taille ≤ 5 µm pour fournir la composition de poudre comprenant une poudre activée par voie mécano-chimique de silice colloïdale mélangée avec du chlorure de sodium en poudre.

5. Procédé de production d'une composition de poudre pour la préparation de solutions pour perfusion de préparations antimicrobiennes, ledit procédé incluant le mélange de dextrose avec d'autres composants, **caractérisé en ce que** le dextrose sous forme de poudre est mélangé avec une silice colloïdale de type poudre en un rapport pondéral du dextrose à la silice colloïdale de 20/1 à 20/5 ou de 50/1 à 50/5, respectivement, et le mélange obtenu est activé mécaniquement par choc et action abrasive jusqu'à ce que la fraction massique des particules finement dispersées de silice colloïdale soit augmentée au moins par deux, comprenant ainsi au moins 35 % de particules de silice colloïdale d'une taille ≤ 5 µm pour fournir la composition de poudre comprenant une poudre activée par voie mécano-chimique de silice colloïdale mélangée avec le dextrose en poudre.

6. Procédé de production d'une composition de poudre pour la préparation de solutions pour perfusion de préparations antimicrobiennes selon les revendications 4 ou 5, **caractérisé en ce que** la silice colloïdale est constituée de nanoparticules de silice ayant un diamètre moyen de 7 nm à 40 nm regroupées en microparticules ayant une taille inférieure à 100 µm.

7. Procédé de préparation d'une solution pour perfusion de préparations antimicrobiennes utilisant une composition de poudre comprenant du chlorure de sodium en poudre ou du dextrose en poudre selon l'une quelconque des revendications 1 à 3, **caractérisé en ce que** le procédé comprend :
- le choix d'un agent antimicrobien à injecter à un mammifère, étant l'agent antimicrobien sous une forme anhydre ;
- la dissolution de l'agent antimicrobien dans de l'eau stérile pour fournir une solution antimicrobienne ;
- le mélange de la solution antimicrobienne avec la composition de poudre par secouage intense pour fournir une solution antimicrobienne pour perfusion ; et
- la dilution de la solution antimicrobienne pour perfusion préparée soit dans 50 à 200 ml de solution de chlorure de sodium à 0,45 % ou 0,9 %, respectivement, soit dans 50 à 200 ml de solution de dextrose à 2 % ou 5 %, respectivement, en fonction de la composition de poudre comprenant le chlorure de sodium en poudre ou le dextrose en poudre, respectivement, pour fournir une préparation de solution antimicrobienne pour perfusion prête à l'emploi.

8. Procédé selon la revendication 7, **caractérisé en ce que** la préparation de solution antimicrobienne pour perfusion prête à l'emploi comprend au moins 40 % de l'agent antimicrobien sorbé au niveau de la poudre activée par voie mécano-chimique de silice colloïdale.
